Europäisches Patentamt

European Patent Office

Office européen des brevets

(19)

(11) Publication number: **0 325 398**
A1

# EUROPEAN PATENT APPLICATION

(12)

(21) Application number: **89300382.2**

(22) Date of filing: **17.01.89**

(51) Int. Cl.4: **G 01 N 33/52**

(30) Priority: **18.01.88 JP 8073/88**

(43) Date of publication of application:
**26.07.89 Bulletin 89/30**

(84) Designated Contracting States: **DE GB**

(71) Applicant: **KONICA CORPORATION**
**26-2 Nishishinjuku 1 chome**
**Shinjuku-ku Tokyo (JP)**

(72) Inventor: **Fukazawa, Koji**
**Konica Corporation 1 Sakura machi**
**Hino-shi Tokyo (JP)**

**Suzuki, Yasuyuki**
**Konica Corporation 1 Sakura machi**
**Hino-shi Tokyo (JP)**

**Kobayashi, Shigeru**
**Konica Corporation 1 Sakura machi**
**Hino-shi Tokyo (JP)**

(74) Representative: **Ellis-Jones, Patrick George Armine et al**
**J.A. KEMP & CO. 14 South Square Gray's Inn**
**London WC1R 5EU (GB)**

(54) **Biochemical analytical element.**

(57) An analytical element which having on a support plural number of layers including a reagent layer and a spreading layer in lamination, wherein at least one of the reagent layer and spreading layer is coated in a predetermined pattern, and a method for manufacturing the same are disclosed.

EP 0 325 398 A1

Bundesdruckerei Berlin

**Description**

## BIOCHEMICAL ANALYTICAL ELEMENT

Field of the invention

This invention relates to an analytical element such as a blood analytical element which is used to identify the content of a certain component in a liquid sample such as blood, serum, etc., or to determine the content of a certain element therein by using a chemical method, or an immune analytical element in which an immunological reaction is utilized.

Background of the invention

Generally, this type of analytical element comprises at least a reagent layer and a spreading layer coated on or adhered to a support, which may further comprise, depending upon the necessity, an adhesive layer, an intermediate layer or a buffer layer. Typical examples of the layer structure of such analytical elements are shown in the drawing. Analytical elements are usually prepared by, for example, cutting a large sheet with the above-mentioned configuration into small pieces having 14 mm X 14 mm, or 16 mm X 16 mm in size. Thus the reagent and spreading layers are usually coated on the whole surface area of the support.

Measurement with this analytical element involves diffusion and distribution of a small quantity (about 10 to 20 $\mu\ell$) of a dripped liquid sample at a constant amount per constant area (about 10 mm$\varphi$) in the reagent layer, and reaction with the reagent to change color, the process and the result of the color change in the reagent layer being optically measured through the support. It is not only unnecessary for the reagent and the spreading layers to be provided on the whole area of the support, but also costly especially when the coating liquid containing the reagent and the spreading layers are expensive.

The analytical element thus obtained by the conventional method has a tendency of causing an orientation of the fiber in the spreading layer when the spreading layer is coated. Thus there is a problem that a dripped liquid sample spreads more in the orientation direction and less in the orthogonal direction. Accordingly, the sample does not circularly diffuse and the photometric area has to be smaller.

Summary of the invention

The object of this invention is to solve the above problems, and to provide an analytical element which enables reduction in loss of the coating liquid when the reagent and spreading layers are formed, and eliminates orientation of the fiber in a certain direction in the case where a coating liquid with fiber dispersion is coated.

The present invention has been made for the purpose of attaining the object and relates to an analytical element which comprises a support and provided thereon plural number of layers, preferably including a reagent layer and a spreading layer, in lamination, wherein at least one of said layers has been provided by coating in a predetermined pattern.

Brief description of the drawings

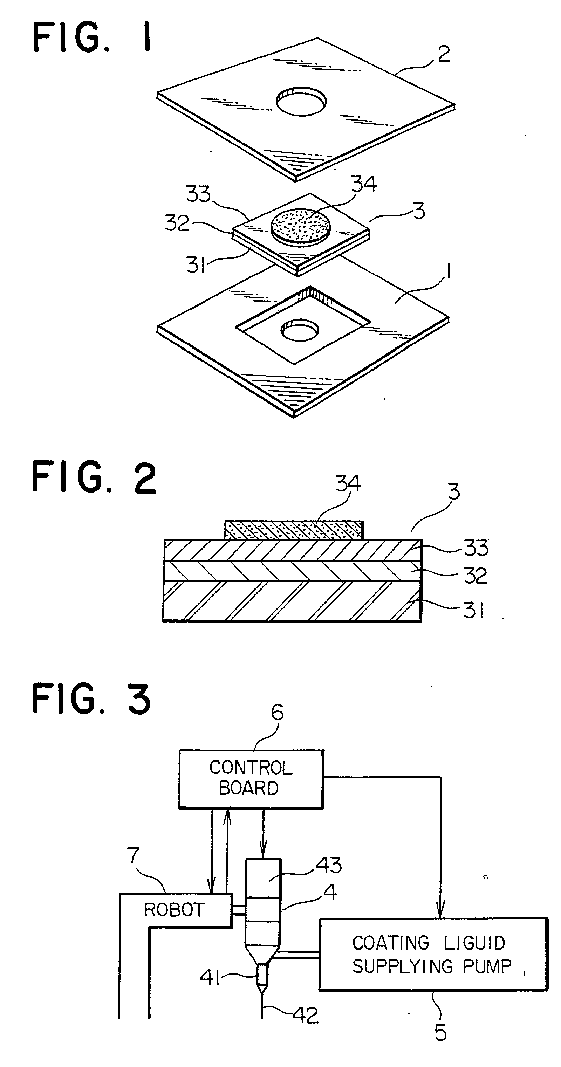

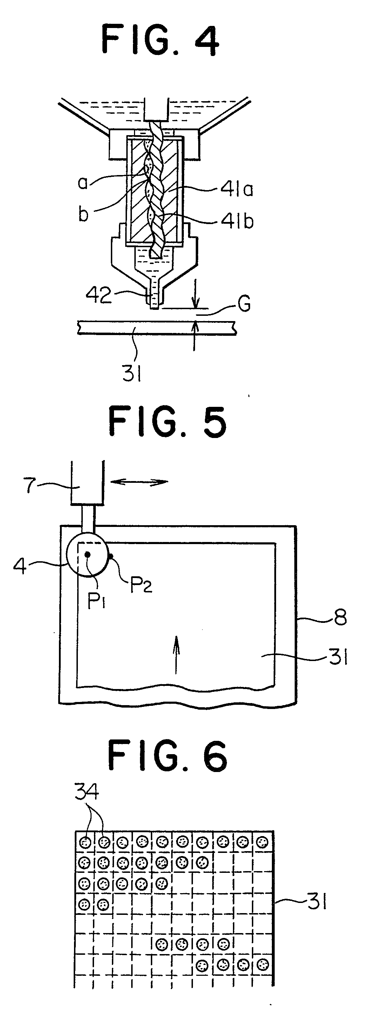

Fig. 1 shows a perspective view showing the relative arrangement of the analytical element, the mount base and the mount cover. Fig. 2 is an enlarged sectional view of the analytical element. Fig. 3 shows a brief sectional view of a pattern coating apparatus. Fig. 4 is an enlarged sectional view of a Mohno-pump. Fig. 5 shows a drawing to explain the arrangement during pattern coating. Fig. 6 presents cutting lines after coating. In the drawings, respective numerals denote as follows:

    3... Analytical element
    31.. Support
    32.. Adhesive layer
    33. Reagent layer
    34. Spreading layer
    4... Pattern coating apparatus

Embodiments of the invention

Hereinafter, the present invention will be explained with reference to the drawings, which illustrate preferable embodiments of the present invention.

In Fig. 1, numeral 1 denotes a mount base, 2 a mount cover and 3 an analytical element provided between said mount base 1 and said mount cover 2. The analytical element 3 shown in Fig. 2 comprises, on the surface of a transparent support 31, an adhesive layer 32, a reagent layer 33 and a spreading layer 34, respectively provided by coating.

The spreading layer 34 causes the dripped liquid sample to diffuse horizontally and to move into the reagent layer 33, in which reaction takes place. In the present invention, pattern coating apparatus 4 shown in Fig. 3 may preferably be utilised for the reasons that the sample liquid may contain very expensive material (for example, avidin immobilized powdery filter paper D) even though their quantities are very small, and that adequate quantity of the sample liquid would be that which can cause diffusion thereof with a size of about 10 mm in diameter.

The type of the pattern coating apparatus 4 is not limited to that described in these examples, however, a preferable model would be one as shown in Fig. 3, in which the coating liquid supplied from supplying pump 5 is deliverd out from nozzle 42 by the use of Mohno-pump 41. The Mohno-pump 41 is provided with a spiral rotor 41b in a cylinder 41a having repeated constriction portion a and inflated portion b in accord with rotation of the spiral rotor in the direction of the axis thereof as shown in Fig. 4. This spiral rotor 41b, shown in Fig. 3, is driven by a motor 43 which is controlled by a signal from a control board 6. By the use of Mohno-pump 41, the amount of the coating liquid can freely be controlled by varying the rotation angle of the rotor 41b, avoiding excess amount of supply of the coating liquid.

Further, with the Mohno-pump, is possible to suck back excess coating liquid pushed out from the nozzle 42 by reversely rotating the rotor 41b, to avoid dripping of the coating liquid. Thus, by using this coating apparatus in a fixed position and making the coating liquid push forward from the nozzle, it is possible to coat a liquid in dots. In this case, the diameter of the dot becomes larger or smaller depending on the quantity of the liquid. It is also possible to draw a straight line or circles if the coating apparatus is connected with a robot 7. As a pattern coating apparatus, a dispenser with a pressure controlling system, which is available on the market may also be used to achieve the object of the present invention.

Next, a coating method of the spreading layer 34 coated by the aforementioned pattern coating apparatus will be explained.

The adhesive and reagent layers are coated over the whole surface of a 500 X 1000 mm support by using coating apparatus such as a blade coater, etc. After drying these layers, the support is placed on a horizontally movable board 8 shown in Fig. 5. Then, the above pattern coating apparatus 4 is connected to a robot 7 so that it may be movable in both right and left directions. Then, after setting the position of the nozzle 42 at first point $P_1$, and the gap between the reagent coated support and the nozzle 42, the rotor 41b is rotated by control board 6 (The quantity delivered is controlled by the rotating angle of the rotor 41b) to deliver the necessary quantity of coating liquid through the nozzle 42. In this case, the liquid may be coated on the support either in dots by setting the nozzle to the foregoing point or in a manner so as to draw a circle by operating the robot.

Thus the pattern coating at the first point $P_1$ is finished. In this case, a 14 mm gap has been used, but other dimensions are acceptable. Thereafter the pattern coating apparatus is shifted by 14 mm by the robot 7, so that the nozzle is at the point $P_2$. The reason why the distance of the shift is set at 14 mm in this example was because the size of the biochemical analytical element was set to be 14 mm x 14 mm and, therefore, this shifting distance may be varied depending on the design.

After the pattern coating of the first lateral line on the support is completed, the board is longitudinally shifted by 14 mm to carry out coating of the second line in the same manner, from the first point of the second line. In like manner, coating of the whole can be completed.

After drying the coating, the support is cut along the broken lines shown in Fig. 6, so that the pattern coated portion is positioned in the center.

Thus the biochemical analytical element bearing a required coated pattern can be obtained.

Experimental Examples

| Support: | 180μ thick polyethylene terephthalate (PET) substrate with an undercoat | |
|---|---|---|
| Coating liquid compositions: | | |
| First layer | Gelatin | 6.0 g |
| | 20 % Tri Ton X-100 (Product of Rohm & Hass) | 3.0 g |
| | Water | 51.0 g |
| Second layer | 20 % Tri Ton X-100 | 0.30 g |
| | n-BuOH | 56.20 g |
| | Vinylpyrrolidone/vinylacetate copolymer | 2.40 g |
| Third layer | 20 % tri ton X-100 | 1.50 g |
| | n-BuOH | 56.55 g |
| | polyvinylpyrrolidone | 1.50 g |
| | powdery filter paper D (product of Toyo Roshi Co.) | 15.00 g |

The first and second layers were coated by the blade coater onto the whole surface of the support and after drying these layers, the third layer was coated by using a pattern coating apparatus 4 with a Mohno-pump and a rotation speed of 60 rpm.

With a distance between the coated support and the nozzle of 0.7 mm and by giving the rotor 41 a half turn, 0.06 cc of the coating liquid was applied on the coated support. The coating liquid thus applied was diffused to form a circle of 14 mmφ with even thickness.

Next, the coating apparatus 4 was connected with a robot, and under similar conditions the coating liquid was applied to the coated support so as to draw a circle of 5 mm diameter. The coating liquid thus applied diffused towards the inside and outside of the circle on the coated support, to form a circular coating having 15 mmφ with even thickness.

The results proved that by using pattern coating apparatus 4 it is possible to obtain a pattern coating with even thickness, by means of either a dotted coating or a coating by drawing a circle. Also, the results have proved that no substantial orientatin of the fiber contained in the coating liquid is observed because the coating liquid diffuses outwards from a central point or outwards/inwards from a central circle.

Ten analytical elements having on a support a reagent layer prepared by the conventional method and a spreading layer were prepared by each of the following three methods, i.e.,

(I) a spreading layer having a diameter of 14 mm was formed from a dot by using the pattern coating apparatus as mentioned hereinbefore;

(II) the spreading layer having a diameter of 15 mm was formed from a drawn circle of 5 mm diameter as mentioned hereinbefore; and

(III) the spreading layer was prepared by the conventional method i.e., the entire surface of the coated support was coated by using a blade coater.

Then 10 μℓ of an aqueous red-dye solution was dripped on these three kinds of elements, and after seven minutes, diameters(longitudinal and lateral directions) of the spots formed by the dye were measured, to obtain results given in the following table.

TABLE

| Analytical Element | Spot Diameter | |
|---|---|---|
| | Average Length (Longitudinal) | Average Length (Lateral) |
| I | 10.3 mm | 10.4 mm |
| II | 10.5 mm | 10.4 mm |
| III | 10.4 mm | 9.6 mm |

It is apparent from the table that the analytical elements in accordance with the present invention (Elements I and II), in comparison with the conventional biochemical analytical element (Element III), show little difference between longitudinal and lateral lengths, being nearly pure circles. On the other hand, in the case of the conventional elements the spots formed by the dye were eliptical.

In this example, only the third layer (spreading layer) was pattern coated, but it is also possible that the first layer and/or the second layer can be coated in the same manner, which is specially advantageous when an expensive material is used in the reagent layer.

As mentioned above, the invention is characterized in that the analytical element comprising on a support a reagent layer and a spreading layer in lamination, with either one or more of these layers pattern coated, and thus it becomes possible to reduce costs for the manufacture and substantially eliminate the orientation of the fiber contained in the coating liquid, which had often accompanied the analytical element prepared by the conventional coating method.

The analytical element of the present invention can be modified in various ways. For example, layer structure of the element may be modified to have an intermediate layer between the support and the reagent layer, or between the reagent layer and the spreading layer, or to have more than two reagent layers and/or spreading layers. Typical examples of the layer structure are as follows, in order from the support:

(i) Support - Reagent Layer - Spreading Layer

(ii) Support - Reagent Layer - Adhesion Layer -Spreading Layer

(iii) Support - Reagent Layer - Buffer Layer - Spreading Layer

(iv) Support - 1st Reagent Layer - 2nd Reagent Layer -Adhesion Layer or Buffer Layer - Spreading Layer

(v) Support - 1st Reagent Layer - 2nd Reagent Layer -Adhesion Layer or Buffer Layer - 1st Spreading Layer - 2nd Spreading Layer

Claims

1. An analytical element which comprises a support and provided thereon plural number of layers, wherein at least one of said layer has been coated in a predetermined pattern.

2. The analytical element of claim 1, wherein said layers comprises a reagent layer and a spreading layer.

3. The analytical element of claim 1, wherein said predetermined pattern is a circle.

4. The analytical element of claim 2, wherein said layers comprises said reagent layer and said spreading layer in this order from said support, and further comprises an adhesion layer between said support and said reagent layer.

5. The analytical element of claim 1, wherein said predetermined pattern is formed by using a pattern coating apparatus.

6. A method of manufacturing an analytical element which comprises on a support plural number of layers including a reagent layer and a spreading layer in lamination, comprising a step of forming at least one of said reagent layer and said spreading layer by coating in a predetermined pattern.

7. The method of claim 5, wherein said predetermined pattern is a circle.

8. The method of claim 5, wherein said predetermined pattern is formed using a pattern coating apparatus.

# FIG. 1

2

33
34
32
3
31

1

# FIG. 2

34
3
33
32
31

# FIG. 3

6

CONTROL
BOARD

7

43
4

ROBOT

41

42

COATING LIGUID
SUPPLYING PUMP

5

# FIG. 4

a
b
41a
41b
42
G
31

# FIG. 5

7
4
P1
P2
8
31

# FIG. 6

34
31

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 066 648 (FUJI PHOTO FILM CO., LTD) * Page 10, lines 9-23; page 20, line 23 - page 21, line 22; page 32, line 9 - page 33, line 6; page 42, lines 14-20; figures 2-4 * | 1-4,6,7 | G 01 N 33/52 |
| X | US-A-4 248 829 (MASAO KITAJIMA et al.) * Figures 1,4; column 4, line 14 - column 5, line 2; column 14, lines 32-39; column 6, lines 4-12 * | 1-3,6,7 | |
| Y | DE-A-3 133 538 (FUJI PHOTO FILM CO., LTD) * Whole document * | 1-3,6,7 | |
| Y | US-A-4 042 335 (P.L. CELMENT) * Column 8, line 50 - column 10, line 16; column 12, line 44 - column 16, line 52 * | 1-3,6,7 | |
| | | | **TECHNICAL FIELDS SEARCHED (Int. Cl.4)** |
| | | | G 01 N |

The present search report has been drawn up for all claims

| Place of search | Date of completion of the search | Examiner |
|---|---|---|
| THE HAGUE | 08-05-1989 | HITCHEN C.E. |